# FASCICULE DE BREVET EUROPEEN

(11) **EP 1 592 659 B1**
(45) Date de publication et mention de la délivrance du brevet: **22.07.2009**
(21) Numéro de dépôt: 04704260.1
(22) Date de dépôt: 22.01.2004
(51) Int. Cl.: C07C 253/10, C07C 253/34

(54) **PROCEDE DE FABRICATION DE COMPOSES DINITRILES**
VERFAHREN ZUR HERSTELLUNG VON DINITRILEN
METHOD OF PRODUCING DINITRILE COMPOUNDS

(30) Priorité: 10.02.2003 FR 0301529
(43) Date de publication de la demande: 09.11.2005
(73) Titulaire: Invista Technologies S.à.r.l., 9000 St. Gallen (CH)
(72) Inventeur: ROSIER, Cécile, F-69510 Soucieu en Jarrest (FR); KABIR, Hocine, F-69360 Serezin du Rhone (FR); MARION, Philippe, F-69390 Vernaison (FR)
(74) Mandataire: Cockerton, Bruce Roger
(86) Numéro de dépôt international: PCT/FR2004/000143
(87) Numéro de publication internationale: WO 2004/080924

(56) Documents cités:
- WO-A-99/06356
- GB-A- 2 212 155
- US-A- 3 766 241
- US-B1- 6 207 851

## Description

La présente invention concerne un procédé de fabrication de composés comprenant deux fonctions nitriles.

Elle se rapporte plus particulièrement à un procédé de fabrication de composés dinitriles à partir de composés comprenant une fonction nitrile et une insaturation éthylénique.

Les composés dinitriles, notamment l'adiponitrile est un intermédiaire chimique important pour la synthèse de nombreux composés. Ainsi, l'adiponitrile est utilisé pour la fabrication d'hexaméthylène diamine, un des monomères des polyamides. Il peut également être utilisé pour la fabrication de l'aminocapronitrile ou du caprolactame, monomères importants pour la production de différents polyamides.

Parmi les procédés proposés pour la synthèse de l'adiponitrile, le procédé utilisant le butadiène comme matière première et la réaction d'hydrocyanation avec du cyanure d'hydrogène est le plus exploité d'un point de vue industriel.

Ce procédé consiste dans une première étape à réaliser l'hydrocyanation d'une oléfine telle que le butadiène pour produire des composés comprenant une fonction nitrile et une insaturation éthylénique. Cette étape est généralement réalisée en présence d'un système catalytique comprenant un complexe organométallique formé par un métal tel que le nickel et un ligand organophosphoré.
Après séparation des composés mononitriles insaturés et éventuellement purification, ceux-ci sont transformés en composés dinitriles par une seconde réaction d'hydrocyanation réalisée également en présence d'un système catalytique comprenant un complexe organométallique formé par un métal tel que le nickel et un ligand organophosphoré. De plus, le système catalytique comprend un cocatalyseur consistant généralement en un acide de Lewis.

Par acide de Lewis, on entend, selon la définition usuelle, des composés accepteurs de doublets électroniques. Les acides de Lewis sont généralement des sels d'éléments métalliques comme cela est décrit ci-après.

Dans les procédés actuels, l'acide de Lewis est maintenu dans le milieu réactionnel notamment pendant l'étape d'extraction des nitriles. L'acide de Lewis est ensuite éliminé avec le pied de distillation, notamment quand il n'est pas séparé du milieu conjointement avec le complexe organométallique utilisé comme système catalytique.

La présence de l'acide de Lewis lors de la distillation du dinitrile peut favoriser la génération d'impuretés dans le milieu, impuretés qui peuvent être présentes dans le dinitrile distillé. En outre, l'élimination de l'acide Lewis et son rejet comme effluent peuvent être préjudiciables à l'économie du procédé et à l'environnement. Un des buts de la présente invention est de proposer un procédé de fabrication de composés dinitriles ne comprenant pas ces inconvénients.
A cet effet, l'invention propose un procédé de fabrication de composés dinitriles par hydrocyanation avec du cyanure d'hydrogène de composés comprenant une fonction nitrile et une insaturation éthylénique en présence d'un système catalytique comprenant un complexe organométallique et un cocatalyseur, formé par un composé métallique.
Selon l'invention, le procédé comprend les étapes successives suivantes, après avoir réalisée l'étape d'hydrocyanation :
I. Traiter le milieu réactionnel E₁ obtenu après l'étape d'hydrocyanation des composés nitriles insaturés pour extraire au moins le complexe organométallique dudit milieu et obtenir un second milieu E₂;
II. Traiter ledit second milieu E₂ par passage sur une résine échangeuse d'ions pour extraire au moins le métal formant le cocatalyseur et obtenir un troisième milieu E₃, et
III. Séparer les dinitriles formés dudit milieu E₃,
et en ce que l'ordre des étapes II); III) peut être inversé.

Selon une caractéristique préférée de l'invention, le cocatalyseur est un acide de Lewis Plus précisément, les acides de Lewis cités dans l'ouvrage édité par G.A. OLAH "Friedel-Crafts and related Reactions", tome 1 pages 191 à 197 (1953) sont convenables pour l'invention.

Les acides de Lewis qui peuvent être mis en oeuvre comme cocatalyseurs dans le présent procédé sont choisis parmi les composés des éléments des groupes Ib, IIb, IIIa, IIIb, IVa, IVb, Va, Vb, Vlb, Vllb et VIII de la Classification périodique des éléments. Ces composés sont le plus souvent des sels, notamment des halogénures, comme chlorures ou bromures, sulfates, sulfonates, halogénoalkylsulfonates, perhalogénoalkylsulfonates, notamment fluoroalkylsulfonates ou perfluoroalkylsulfonates, halogénoacétates, perhalogénoacétates, carboxylates et phosphates.

A titre d'exemples non limitatifs de tels acides de Lewis, on peut citer le chlorure de zinc, le bromure de zinc, l'iodure de zinc, le chlorure de manganèse, le bromure de manganèse, le chlorure de cadmium, le bromure de cadmium, le chlorure stanneux, le bromure stanneux, le sulfate stanneux, le tartrate stanneux, le chlorure d'indium, le trifluorométhylsulfonate d'indium, le trifluoroacétate d'indium, les chlorures ou bromures des éléments des terres rares comme le lanthane, le cérium, le praséodyme, le néodyme, le samarium, l'europium, le gadolinium, le terbium, le dysprosium, l'hafnium, l'erbium, le thallium, l'ytterbium et le lutétium, le chlorure de cobalt, le chlorure ferreux, le chlorure d'yttrium.

On peut également utiliser comme acide de Lewis des composés comme le triphénylborane, l'isopropylate de titane.

On peut bien entendu mettre en oeuvre des mélanges de plusieurs acides de Lewis.
Parmi les acides de Lewis, on préfère tout particulièrement le chlorure de zinc, le bromure de zinc, le chlorure stanneux, le bromure stanneux le triphénylborane le trifluorométhylsulfonate d'indium, le trifluoroacétate d'indium, et les mélanges chlorure de zinc/chlorure stanneux.
Le cocatalyseur acide de Lewis mis en oeuvre représente généralement de 0,01 à 50 moles par mole de composé de métal de transition, plus particulièrement de composé du nickel.
Selon un mode de réalisation préféré de l'invention, l'étape **II**) de traitement sur résines échangeuses d'ions est mise en oeuvre avant la séparation des composés dinitriles du milieu E₃.

En effet, la séparation des composés dinitriles est généralement réalisée par distillation donc chauffage du milieu E₃ contenant ces composés. Il est avantageux d'éliminer les éléments métalliques contenus dans le milieu E₃ avant de réaliser un tel chauffage pour éviter que ceux-ci ne favorisent des réactions parasites ou une décomposition des dinitriles. Ces réactions parasites ou décomposition génèrent des sous-produits de structure voisine de celle des dinitriles, difficilement séparables de ceux-ci.

Ainsi, dans le cas de l'adiponitrile, certaines impuretés telles que l'imino-1-cyano-2-cyclopentène (ICCP) peuvent être produites. Ces impuretés se retrouvent, en partie, dans l'adiponitrile distillé. Les impuretés et leurs produits d'hydrogénation peuvent également se retrouver dans l'hexaméthylène diamine (HMD) obtenue par hydrogénation de cet adiponitrile, et même dans le polyamide obtenu à partir de l'HMD.

Ainsi, ces impuretés peuvent engendrer des anomalies dans les procédés de mise en forme de ces polyamides, notamment dans les procédés de filage (augmentation du nombre de casse des fils), ou sur la stabilité et la couleur du polyamide.

Selon une caractéristique préférée de l'invention, le complexe organométallique formant le système catalytique est généralement un complexe de coordination entre un élément métallique choisi parmi les métaux de transition et des ligands généralement des ligands organophosphorés.

De tels complexes organométalliques sont décrits dans de nombreuses publications et nombreux brevets tels que US3496215, DE19953058, FR1529134, FR2069411, US3631191, US3766231, FR2523974, WO9906355, WO9906356, WO9906357, WO9906358, WO9952632, WO9965506, WO9962855, US5693843, WO961182, WO9622968, US5981772, WO0136429, WO9964155, WO0213964.
Les éléments métalliques sont généralement choisis dans le groupe comprenant le nickel, le cobalt, le fer, le ruthénium, le rhodium, le palladium, l'osmium, l'iridium, le platine, le cuivre, l'argent, l'or, le zinc, le cadmium, le mercure. Parmi ces métaux le nickel est le métal préféré.

Dans ces complexes métalliques, le métal se retrouve à un degré d'oxydation particulier, notamment zéro pour le nickel, par exemple.

Au cours de la réaction d'hydrocyanation, une partie de l'élément métallique (nickel, par exemple) peut être oxydé à un degré d'oxydation supérieur, degré 1 ou 2, par exemple et donc ne plus être extractible du milieu réactionnel E₁ pendant l'étape I) du procédé de l'invention. Sa présence dans les milieux réactionnels E₂ ou E₃ peut générer des inconvénients semblables à ceux des éléments métalliques provenant du cocatalyseur.

Cet inconvénient est supprimé par le procédé de l'invention qui permet, selon une caractéristique préférée, de co-extraire ces éléments métalliques au cours de l'étape (II) de traitement sur résines échangeuses d'ions en choisissant une résine et des conditions d'extraction appropriées. Il est également possible, sans sortir du cadre de la présente invention d'utiliser deux ou plusieurs résines en combinaison ou successivement pour permettre l'extraction de ces éléments métalliques de manière concomitante ou successive.

Comme complexes organométalliques convenables pour l'invention on peut citer ceux obtenus à partir de composés de nickel et de composés organophosphorés appartenant aux familles des organophosphites, organophosphinites, organophosphonites et organophosphines monodentates ou pluridentates. Il est également possible d'utiliser des complexes organométalliques obtenus à partir de stilbine ou arsine en association avec du nickel ou un des métaux cités précédemment. Des exemples de composés organophosphorés sont décrits dans de nombreuses publications et nombreux brevets. On peut citer par exemple comme exemple de composés monodentates le triphénylphosphite, le tritolylphosphite, le trithymolphosphite, le triphénylphosphinite, le tritolylphosphinite, le trithymolphosphinite, le triphénylphosphonite, le tritolylphosphonite, la trithymolphosphonite, la triphénylphosphine, la tritolylphosphine, la trithymolphosphine.

Comme composés bidentates, on peut citer, à titre d'exemple, les structures suivantes dans lesquelles Ph signifie phényle :

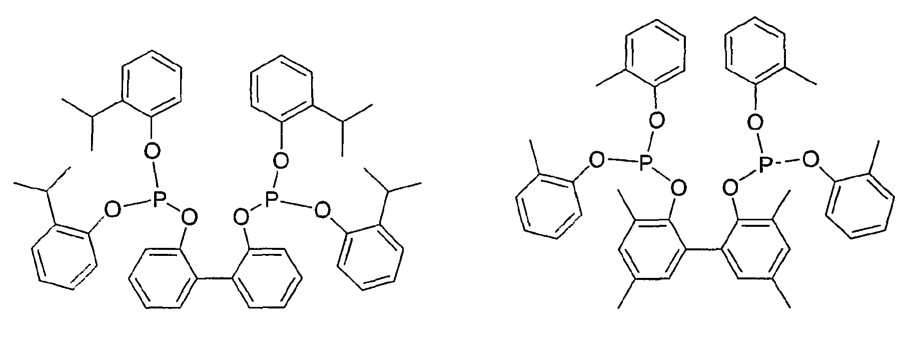

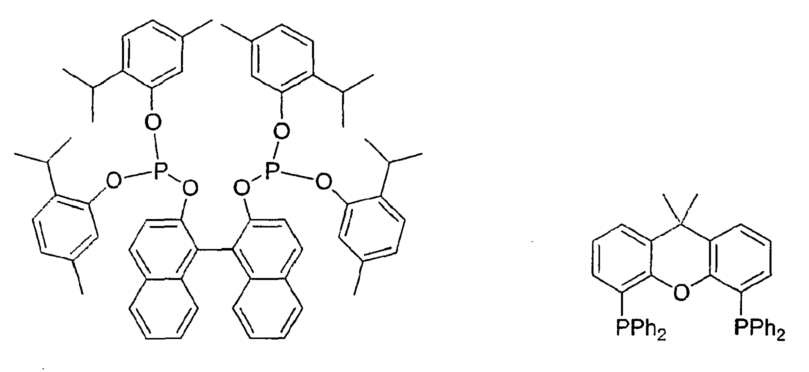

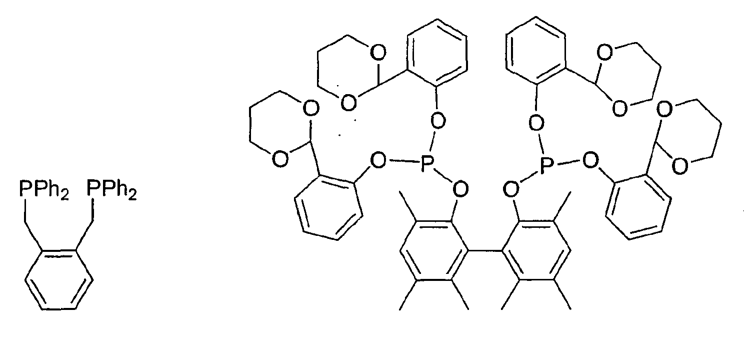

Plus généralement, tous les ligands organophosphorés sont convenables pour l'invention.

A titre d'exemple, on peut également citer les ligands et systèmes catalytiques décrits dans les brevets WO 95/30680, WO 96/11182, WO 99/06358, WO 99/13983, WO 99/64155, WO 01/21579, WO 01/21580.

Les complexes organométalliques peuvent être préparés avant leur addition dans le milieu réactionnel ou in-situ.

La préparation des complexes organométalliques peut être effectuée en mettant en contact un composé du métal choisi avec une solution d'un composé organophosphoré.
Le composé du métal peut être dissout dans un solvant.
Le métal peut se trouver dans le composé mis en oeuvre, soit au degré d'oxydation qu'il aura dans le complexe organométallique, soit à un degré d'oxydation supérieur.

A titre d'exemple, on peut indiquer que dans les complexes organométalliques de l'invention, le rhodium est au degré d'oxydation (I), le ruthénium au degré d'oxydation (II), le platine au degré d'oxydation (0), le palladium au degré d'oxydation (0), l'osmium au degré d'oxydation (II), l'iridium au degré d'oxydation (I), le nickel au degré d'oxydation (0).
Si lors de la préparation du complexe organométallique, le métal est mis en oeuvre à un degré d'oxydation plus élevé, il pourra être réduit in situ.

On peut citer à titre d'exemple non limitatif de composés de métal utilisés pour la préparation de ces complexes, les poudres métalliques comme la poudre de nickel et les composés suivants :
- les composés dans lesquels le nickel est au degré d'oxydation zéro comme le tétracyanonickelate de potassium K₄ [Ni(CN)₄], le bis (acrylonitrile) nickel zéro, le bis (cyclooctadiène-1,5) nickel (appelé également Ni(cod)₂ ) et les dérivés contenant des ligands comme le tétrakis (triphényl phosphine) nickel zéro.
- les composés du nickel comme les carboxylates (notamment l'acétate), carbonate, bicarbonate, borate, bromure, chlorure, citrate, thiocyanate, cyanure, formiate, hydroxyde, hydrophosphite, phosphite, phosphate et dérivés, iodure, nitrate, sulfate, sulfite, aryl- et alkylsulfonates.

Quand le composé du nickel utilisé correspond à un état d'oxydation du nickel supérieur à 0, on ajoute au milieu réactionnel un réducteur du nickel réagissant préférentiellement avec celui-ci dans les conditions de la réaction. Ce réducteur peut être organique ou minéral. On peut citer comme exemples non limitatifs les borohydrures comme le BH₄Na, le BH₄K, la poudre de Zn, le magnésium ou l'hydrogène.

Quand le composé du nickel utilisé correspond à l'état d'oxydation 0 du nickel, on peut également ajouter un réducteur du type de ceux précités, mais cet ajout n'est pas impératif.
Quand on utilise un composé du fer, les mêmes réducteurs conviennent.
Dans le cas du palladium, les réducteurs peuvent être, en outre, des éléments du milieu réactionnel (phosphine, solvant, oléfine).

Le cocatalyseur ou acide de Lewis est présent dans le système catalytique selon une quantité comprise entre 0,01 à 50 moles d'acide de Lewis par mole d'élément métallique du complexe organométallique, comme le nickel par exemple, et de préférence entre 0,05 et 10 mole/mole. L'acide de Lewis peut être ajouté directement dans le milieu réactionnel ou avec le complexe organométallique.

La réaction d'hydrocyanation est généralement mise en oeuvre à une température comprise entre 10°C et 200 °C, de préférence entre 30 °C et 120 °C.
La réaction d'hydrocyanation peut être conduite sans solvant, mais il peut être avantageux d'ajouter un solvant organique inerte. Ce solvant peut être un solvant du système catalytique qui est miscible à la phase ou au milieu comprenant le composé à hydrocyaner, au moins à la température d'hydrocyanation.

Cette réaction ainsi que le procédé de l'invention peut être réalisé de manière continue ou discontinue.

Cette réaction peut être également réalisée en présence d'un système biphasique comprenant notamment une phase aqueuse dans laquelle le complexe organométallique est soluble. Dans ce mode de réalisation, la phase aqueuse est séparée avec la plus grande partie du complexe organométallique en fin de réaction, la phase organique contenant les dinitriles et le nitriles insaturés qui n'ont pas réagi est le milieu E₁ au sens de la présente invention. En effet, il est intéressant de traiter cette phase organique selon le procédé de l'invention pour extraire la faible partie de complexe organique et d'acide de Lewis contenue dans ladite phase.

Le milieu réactionnel E₁ obtenu en sortie du réacteur d'hydrocyanation est, selon un mode de réalisation préférentiel de l'invention, alimenté dans une étape I) d'extraction du complexe organométallique.
Cette étape peut consister en une extraction dudit complexe par un solvant dans une installation d'extraction liquide/liquide. On peut citer, à titre d'exemple, comme solvant d'extraction, les alcanes comprenant de 5 à 9 atomes de carbone tels que le pentane, l'hexane, l'heptane, les cycloalcanes comprenant de 5 à 8 atomes de carbone comme le cylohexane, méthylcyclohexane, cyclooctane, les hydrocarbures halogénés comprenant de 1 à 5 atomes de carbone tels que le chloroforme, le dichloroéthane, le tétrachlorure de carbone, le chloropropane, le dichlorométhane, les composés aromatiques substitués ou non comprenat de 6 à 9 atomes de carbones tels que le benzène, le toluène, le xylène,l'éthylbenzène, l'isopropylbenzène.

Cette séparation peut également être obtenue par distillation des nitriles insaturés et obtention d'un milieu biphasique dont l'une des phases contient le complexe organométallique, l'autre phase contenant principalement les dinitriles. Cette dernière peut être soumise à une extraction liquide/liquide avec les solvants décrits ci-dessus pour extraire les traces de complexe organométallique.

Le complexe organométallique ainsi extrait peut être recyclé dans une réaction d'hydrocyanation. A la fin de l'étape I), un milieu réactionnel E₂ ne comprenant plus de complexe organométallique est obtenu. Par « ne comprend plus » il faut comprendre que le maximum du complexe organométallique a été extrait mais il peut rester des traces dudit complexe dans le milieu sans pour cela sortir du cadre de l'invention

Ce milieu réactionnel E₂ contient les dinitriles formés ainsi que l'acide de Lewis et éventuellement des composés de l'élément métallique provenant du complexe organométallique ayant subi une oxydation ou non.

Selon le mode de réalisation préféré de l'invention, ce milieu réactionnel E₂ est soumis à un traitement sur résines échangeuses d'ions permettant de fixer et extraire les ions métalliques de l'acide de Lewis et éventuellement les ions métalliques oxydés provenant du complexe organométallique et se trouvant dans ledit milieu E₂.

Les résines échangeuses d'ions convenables pour la présente invention sont choisies en fonction de la nature des éléments métalliques à extraire. Ainsi ces résines peuvent être une de celles appartenant au groupe comprenant les résines cationiques fortes ou faibles, les résines adsorbantes, les résines chélatantes, et les résines catalytiques. A titre d'exemples de résines on peut citer les résines sulfoniques, les résines carboxyliques, les résines iminodiacétiques, les résines commercialisées sous les appellations commerciales AMBERLITE et AMBERLYST par la société ROHM & HAAS, DOWEX par la société DOW, LEVATITE ou IONAC par la société BAYER.

Cette étape II) peut être mise en oeuvre dans tous dispositifs connus de l'homme du métier. Ainsi, on peut utiliser des colonnes comprenant ces résines sous forme de lit fixe ou lit fluidisé ou dans des systèmes à membrane formée par ladite résine.

Après traitement sur résine, un milieu réactionnel E₃ est obtenu qui contient essentiellement les composés organiques dinitriles formés. Ces différents composés seront avantageusement séparés dans une étape (III) de distillation. Toutefois, d'autres procédés de séparation pourront être utilisés sans pour cela sortir du cadre de l'invention.
Par ailleurs, l'étape II) de traitement des résines comprend également une étape d'élution des résines chargées avec les éléments métalliques extraits pour ainsi régénérer les résines et récupérer ces éléments métalliques.
Cette élution est une étape classique et usuelle des procédés de traitement sur résines échangeuses d'ions. Elle peut être réalisée notamment en utilisant des acides forts tels que les acides sulfurique, chlorhydrique ou des acides organiques forts et de préférence, l'acide correspondant à l'anion de l'acide de Lewis à régénérer.

Dans le cadre de la présente invention, cette étape d'élution peut permettre de récupérer et régénérer l'acide de Lewis et ainsi permettre sa réutilisation dans une étape d'hydrocyanation. Cette possibilité présente un avantage important pour permettre une exploitation économique du procédé et un respect de l'environnement, notamment quand le composé utilisé comme acide de Lewis est onéreux et/ou présente un caractère nocif pour l'environnement.

Le procédé de l'invention s'applique notamment aux nitriles aliphatiques comprenant une insaturation éthylénique et plus particulièrement les pentènenitriles linéaires comme le pentène-3-nitrile, le pentène-4-nitrile.

Ces pentènenitriles peuvent contenir des quantités minoritaires d'autres composés, comme le méthyl-2--butène-3-nitrile, le méthyl-2-butène-2-nitrile, le pentène-2-nitrile, le valéronitrile, l'adiponitrile, le méthyl-2-glutaronitrile, l'éthyl-2-succinonitrile. Ces composés sont présents dans les pentènenitriles notamment quand ces derniers proviennent d'une première étape d'hydrocyanation du butadiène en mononitriles insaturés.

Cette première étape est généralement réalisée en présence d'un catalyseur comprenant un complexe organométallique mais en absence de cocatalyseur. Le complexe organométallique utilisé dans cette première étape peut être différent ou identique à celui utilisé dans le procédé de l'invention. Dans le cas où il est identique, le complexe organométallique récupéré à l'étape I) du procédé de l'invention peut être recyclé dans le réacteur d'hydrocyanation de la première étape d'hydrocyanation du butadiène.

Les mononitriles introduits dans le procédé de l'invention peuvent également provenir d'une étape d'isomérisation, généralement associée à la première étape ci-dessus, qui consiste à maintenir les composés obtenus à la première étape ci-dessus en présence d'un système catalytique comprenant un complexe organométallique, avantageusement identique à celui de la première étape, en absence de cyanure d'hydrogène.

Cette étape d'isomérisation a pour objectif d'améliorer la sélectivité du procédé en mononitriles insaturés linéaires.

D'autres avantages et détails de l'invention apparaîtront plus clairement au vu des exemples donnés ci-dessous uniquement à titre indicatif et d'illustration.

### Exemple 1 :

On prépare une solution de trifluoroacétate d'indium (0.5g, 1.10mmol) dans un mélange de 3-pentènenitrile (36.4g, 448mol) et adiponitrile (63.6g, 588mmol). La composition du milieu est déterminée par analyse élémentaire.
Des fractions de 4 ml de cette solution sont mises en contact avec 2ml de chacune des résines suivantes commercialisées par le société Rohm & Haas sous les appellations commerciales indiquées ci-dessous:
- une résine sulfonique (Amberlite 252H),
- une résine chélatante (IRC 748),
- une résine adsorbante (Amberlite XAD7)
- et une résine cationique (IRC 50).
Chacun des milieux est agité 4h à Température ambiante (20°C). La résine est séparée par filtration et la composition du filtrat est déterminée par analyse élémentaire.
Les compositions des milieux initiaux et des filtrats sont données dans le tableau I ci-dessous :

| | | **In (ppm)** |
|---|---|---|
| Composition du milieu initial | | 1190 |
| Composition du filtrat | Amberlite 252H Résine sulfonique | Non détectable |
| | IRC 748 Résine chélatante | Non détectable |
| | Amberlite XAD7 Résine adsorbante | Non détectable |
| | IRC 50 Résine cationique | Non détectable |

### Exemple 2 :

Une colonne en verre est remplie de 100ml de résine sulfonique (Amberlite 252H). On alimente en continu par le haut de la colonne une solution de 3-pentènenitrile et d'adiponitrile (30/70 en mole) contenant du trifluoroacétate d'indium de concentration exprimée en élément indium égale à 3300ppm selon un débit de 340g/h. Des échantillons sont prélevés toutes les 10 minutes en sortie de colonne. La concentration en indium dans les échantillons est mesurée par analyse élémentaire.
Pendant les trois heures de fonctionnement de la colonne de séparation, la concentration en résine dans les échantillons a toujours été inférieure à 20 ppm.

### Exemple 3 :

On prépare une solution de chlorure de zinc (0.1g, 0.73mmol) dans un mélange de 3-pentènenitrile (36.4g, 448mmol) et d'adiponitrile (63.6g, 588mmol). La composition du milieu est déterminée par analyse élémentaire.
4 ml de cette solution sont agités pendant 4h à température ambiante (20°C) en présence de 2 ml de résine sulfonique (Amberlite 252H). Une analyse de la solution avant et après traitement sur résine indique que la concentration en Zinc de la solution passe de 480 ppm à 8 ppm.

### Exemple 4 :

On réalise la réaction d'hydrocyanation du 3-pentène-nitrile en adiponitrile catalysée par le système Ni°/ligand organophosphoré/Acide de Lewis(In). Le ligand organophosphoré est le composé de formule suivante :

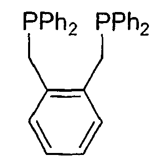

Des fractions de 3ml de ce milieu réactionnel sont agitées pendant 4 heures à température ambiante (20°C environ) en présence de 3ml d'une des résines suivantes :
- une résine sulfonique (Amberlite 252H),
- une résine chélatante (IRC 748),
- une résine adsorbante (Amberlite XAD7)
La composition du milieu réactionnel avant et après traitement sur résine est donnée dans le tableau II ci-dessous :

| | Réf résine | %Ni | %In |
|---|---|---|---|
| Composition initiale | / | 0.459 | 1.0 |
| Composition après traitement | Amberlite 252H | 0.46 | <0.02 |
| | Amberlite XAD-7 | 0.28 | 0.42 |
| | IRC 748 | <0.008 | <0.17 |

Ces essais montrent qu'il est possible d'extraire l'indium provenant de l'acide de Lewis mais également, dans certaines conditions le nickel oxydé provenant du complexe organométallique utilisé comme catalyseur.

## Revendications

1. Procédé de fabrication de composés dinitriles par hydrocyanation de composés mononitriles comprenant une insaturation éthylénique par réaction avec du cyanure d'hydrogène en présence d'un système catalytique comprenant un complexe organométallique et un cocatalyseur **caractérisé en ce qu'**il comprend les étapes successives suivantes :
(I) Traiter le milieu réactionnel E₁ obtenu après hydrocyanation de composés nitriles insaturés pour séparer le complexe organométallique du système catalytique et obtenir un second milieu E₂.
(II) Traiter ledit second milieu E₂ par une résine échangeuse d'ions pour au moins extraire les ions métalliques provenant du cocatalyseur pour obtenir un troisième milieu E₃.
(III) Séparer les dinitriles formés du troisième milieu E₃,
et **en ce que** l'étape (III) peut être réalisée avant l'étape (II).

2. Procédé selon la revendication 1, **caractérisé en ce que** le cocatalyseur est un acide de Lewis.

3. procédé selon la revendication 1 ou 2, **caractérisé en ce que** les ions métalliques extraits à l'étape (II) sont récupérés par élution de la résine.

4. procédé selon la revendication 3, **caractérisé en ce que** les ions métalliques récupérés sont recyclés pour former le cocatalyseur du système catalytique de l'étape d'hydrocyanation.

5. procédé selon l'une des revendications 2 à 4, **caractérisé en ce que** l'acide de Lewis est un composé d'un élément métallique appartenant au groupe Ib, IIIb, IIIb, IVa, IVb, Va, Vb, Vlb, Vllb, VIII de la classification périodique des éléments.

6. Procédé selon l'une des revendications 2 à 5, **caractérisé en ce que** l'acide de Lewis est choisi dans le groupe comprenant des halogénures, comme chlorures ou bromures, sulfates, sulfonates, halogénoalkylsulfonates, perhalogénoalkylsulfonates, notamment fluoroalkylsulfonates ou perfluoroalkylsulfonates, halogénoacétates, perhalogénoacétates, carboxylates et phosphates des éléments métalliques.

7. Procédé selon l'une des revendications 2 à 6, **caractérisé en ce que** l'acide de Lewis est choisi dans le groupe comprenant le chlorure de zinc, le bromure de zinc, l'iodure de zinc, le chlorure de manganèse, le bromure de manganèse, le chlorure de cadmium, le bromure de cadmium, le chlorure stanneux, le bromure stanneux, le sulfate stanneux, le tartrate stanneux, le chlorure d'indium, le trifluorométhylsulfonate d'indium, le trifluoroacétate d'indium, les chlorures ou bromures des éléments des terres rares comme le lanthane, le cérium, le praséodyme, le néodyme, le samarium, l'europium, le gadolinium, le terbium, le dysprosium, l'hafnium, l'erbium, le thallium, l'ytterbium et le lutétium, le chlorure de cobalt, le chlorure ferreux, le chlorure d'yttrium.

8. Procédé selon l'une des revendications précédentes, **caractérisé en ce que** la résine d'extraction des ion métalliques utilisée à l'étape II) est choisie dans le groupe comprenant les les résines cationiques fortes ou faibles, les résines adsorbantes, les résines chélatantes et les résines catalytiques.

9. Procédé selon la revendication 8, **caractérisé en ce que** la résine échangeuse d'ions est choisie dans le groupe comprenant les résines sulfoniques, les résines carboxyliques, les résines iminodiacétiques.

10. Procédé selon l'une des revendications précédentes, **caractérisé en ce que** l'extraction du complexe organométallique à l'étape I) est réalisée par une extraction liquide/liquide avec un solvant d'extraction choisi dans le groupe comprenant les alcanes comprenant de 5 à 9 atomes de carbone, les cycloalcanes comprenant de 5 à 8 atomes de carbone, les hydrocarbures halogénés comprenant de 1 à 5 atomes de carbone, les composés aromatiques substitués ou non comprenant de 6 à 9 atomes de carbones.

11. Procédé selon l'une des revendications précédentes, **caractérisé en ce que** la séparation des dinitriles à l'étape III) est réalisée par distillation desdits dinitriles.

12. Procédé selon l'une des revendication précédentes, **caractérisé en ce que** les composés mononitriles insaturés sont des pentènenitriles.

13. Procédé selon la revendication 12, **caractérisé en ce que** les pentènenitriles sont obtenus par hydrocyanation du butadiène.

## Claims

1. Process for the manufacture of dinitrile compounds by hydrocyanation of mononitrile compounds comprising an ethylenic unsaturation by reaction with hydrogen cyanide in the presence of a catalytic system comprising an organometallic complex and a cocatalyst, **characterized in that** it comprises the following successive stages:
I) treating the reaction medium E₁ obtained after hydrocyanation of unsaturated nitrile compounds in order to separate the organometallic complex from the catalytic system and to obtain a second medium E₂;
II) treating the said second medium E₂ with an ion-exchange resin in order at least to extract the metal ions originating from the cocatalyst in order to obtain a third medium E₃, and
III) separating the dinitriles formed from the third medium E₃,
and **in that** stage III) can be carried out before stage In.

2. Process according to Claim 1, **characterized in that** the cocatalyst is a Lewis acid.

3. Process according to Claim 1 or 2, **characterized in that** the metal ions extracted in stage II) are recovered by elution of the resin.

4. Process according to Claim 3, **characterized in that** the metal ions recovered are recycled in order to form the cocatalyst of the catalytic system of the hydrocyanation stage.

5. Process according to one of Claims 2 to 4, **characterized in that** the Lewis acid is a compound of a metal element belonging to Group Ib, IIIb, IIIb, IVa, IVb, Va, Vb, VIb, VIIb or VIII of the Periodic Table of the Elements.

6. Process according to one of Claims 2 to 5, **characterized in that** the Lewis acid is chosen from the group consisting of halides, such as chlorides or bromides, sulphates, sulphonates, haloalkylsulphonates, perhaloalkylsulphonates, in particular fluoroalkylsulphonates or perfluoroalkylsulphonates, haloacetates, perhaloacetates, carboxylates and phosphates of the metal elements.

7. Process according to one of Claims 2 to 6, **characterized in that** the Lewis acid is chosen from the group consisting of zinc chloride, zinc bromide, zinc iodide, manganese chloride, manganese bromide, cadmium chloride, cadmium bromide, stannous chloride, stannous bromide, stannous sulphate, stannous tartrate, indium chloride, indium trifluoromethylsulphonate, indium trifluoroacetate, the chlorides or bromides of rare earth elements, such as lanthanum, cerium, praseodymium, neodymium, samarium, europium, gadolinium, terbium, dysprosium, hafnium, erbium, thallium, ytterbium and lutetium, cobalt chloride, ferrous chloride and yttrium chloride.

8. Process according to one of the preceding claims, **characterized in that** the resin for extraction of the metal ions used in stage II) is chosen from the group consisting of strong or weak cationic resins, adsorbent resins, chelating resins and catalytic resins.

9. Process according to Claim 8, **characterized in that** the ion-exchange resin is chosen from the group consisting of sulphonic resins, carboxylic resins and iminodiacetic resins.

10. Process according to one of the preceding claims, **characterized in that** the extraction of the organometallic complex in stage I) is carried out by liquid/liquid extraction with an extraction solvent chosen from the group consisting of alkanes comprising from 5 to 9 carbon atoms, cycloalkanes comprising from 5 to 8 carbon atoms, halogenated hydrocarbons comprising from 1 to 5 carbon atoms and substituted or unsubstituted aromatic compounds comprising from 6 to 9 carbon atoms.

11. Process according to one of the preceding claims, **characterized in that** the separation of the dinitriles in stage III) is carried out by distillation of the said dinitriles.

12. Process according to one of the preceding claims, **characterized in that** the unsaturated mononitrile compounds are pentenenitriles.

13. Process according to Claim 12, **characterized in that** the pentenenitriles are obtained by hydrocyanation of butadiene.

## Patentansprüche

1. Verfahren zur Herstellung von Dinitrilverbindungen durch Hydrocyanierung von Mononitrilverbindungen mit einer ethylenischen Ungesättigtheit durch Reaktion mit Cyanwasserstoff in Gegenwart eines katalytischen Systems, das einen metallorganischen Komplex und einen Cokatalysator aufweist, **dadurch gekennzeichnet, daß** das Verfahren die folgenden sukzessiven Phasen aufweist:
I) Behandeln des nach der Hydrocyanierung von ungesättigten Nitrilverbindungen erhaltenen Reaktionsmediums E₁, um den metallorganischen Komplex von dem katalytischen System zu trennen und ein zweites Medium E₂ zu erhalten;
II) Behandeln des zweiten Mediums E₂ mit einem Ionenaustauscherharz, um zumindest die von dem Cokatalysator herrührenden Metallionen zu extrahieren und ein drittes Medium E₃ zu erhalten, und
III) Trennen der gebildeten Dinitrile von dem dritten Medium E₃,
und daß die Phase III) vor der Phase II) ausgeführt werden kann.

2. Verfahren nach Anspruch 1, **dadurch gekennzeichnet, daß** der Cokatalysator eine Lewis-Säure ist.

3. Verfahren nach Anspruch 1 oder 2, **dadurch gekennzeichnet, daß** die in Phase II) extrahierten Metallionen durch Elution des Harzes zurückgewonnen werden.

4. Verfahren nach Anspruch 3, **dadurch gekennzeichnet, daß** die zurückgewonnenen Metallionen im Kreislauf zurückgeführt werden, um den Cokatalysator des katalytischen Systems der Hydrocyanierungsphase zu bilden.

5. Verfahren nach einem der Ansprüche 2 bis 4, **dadurch gekennzeichnet, daß** die Lewis-Säure eine Verbindung eines Metallelements ist, das zu Gruppe Ib, IIIb, IIIb, IVa, IVb, Va, Vb, VIb, VIIb, VIII des Periodensystems der Elemente gehört.

6. Verfahren nach einem der Ansprüche 2 bis 5, **dadurch gekennzeichnet, daß** die Lewis-Säure aus der Gruppe ausgewählt ist, die aus Halogeniden, wie z. B. Chloriden oder Bromiden, Sulfaten, Sulfonaten, Halogenalkylsulfonaten, Perhalogenalkylsulfonaten, insbesondere Fluoralkylsulfonaten oder Perfluoralkylsulfonaten, Halogenacetaten, Perhalogenacetaten, Carboxylaten und Phosphaten der Metallelemente besteht.

7. Verfahren nach einem der Ansprüche 2 bis 6, **dadurch gekennzeichnet, daß** die Lewis-Säure aus der Gruppe ausgewählt ist, die aus Zinkchlorid, Zinkbromid, Zinkiodid, Magnanchlorid, Manganbromid, Cadmiumchlorid, Cadmiumbromid, Zinn(II)-chlorid, Zinn(II)-bromid, Zinn(II)-sulfat, Zinn(II)-tartrat, Indiumchlorid, Indiumtrifluormethylsulfonat, Indiumtrifluoracetat, den Chloriden oder Bromiden von Seltenerdelementen, wie z. B. Lanthan, Cer, Praseodym, Neodym, Samarium, Europium, Gadolinium, Terbium, Dysprosium, Hafnium, Erbium, Thallium, Ytterbium und Lutetium, Cobaltchlorid, Eisen(II)-chlorid und Yttriumchlorid besteht.

8. Verfahren nach einem der vorstehenden Ansprüche, **dadurch gekennzeichnet, daß** das in Phase II) eingesetzte Harz zur Extraktion der Metallionen aus der Gruppe ausgewählt ist, die aus starken oder schwachen kationischen Harzen, Adsorberharzen, chelatbildenden bzw. komplexierenden Harzen und katalytischen Harzen besteht.

9. Verfahren nach Anspruch 8, **dadurch gekennzeichnet, daß** das Ionenaustauscherharz aus der Gruppe ausgewählt ist, die aus Sulfonsäureharzen, Carbonsäureharzen und Iminodiessigsäureharzen besteht.

10. Verfahren nach einem der vorstehenden Ansprüche, **dadurch gekennzeichnet, daß** die Extraktion des metallorganischen Komplexes in Phase I) durch Flüssig-Flüssig-Extraktion mit einem Extraktionsmittel ausgeführt wird, das aus der Gruppe ausgewählt ist, die aus Alkanen mit 5 bis 9 Kohlenstoffatomen, Cycloalkanen mit 5 bis 8 Kohlenstoffatomen, halogenierten Kohlenwasserstoffen mit 1 bis 5 Kohlenstoffatomen und substituierten oder nichtsubstituierten aromatischen Verbindungen mit 6 bis 9 Kohlenstoffatomen besteht.

11. Verfahren nach einem der vorstehenden Ansprüche, **dadurch gekennzeichnet, daß** die Trennung der Dinitrile in Phase III) durch Destillation der Dinitrile ausgeführt wird.

12. Verfahren nach einem der vorstehenden Ansprüche, **dadurch gekennzeichnet, daß** die ungesättigten Mononitrilverbindungen Pentennitrile sind.

13. Verfahren nach Anspruch 12, **dadurch gekennzeichnet, daß** die Pentennitrile durch Hydrocyanierung von Butadien gewonnen werden.
